(19) Europäisches Patentamt
European Patent Office
Office européen des brevets

(11) **EP 0 565 903 B1**

(12) **EUROPÄISCHE PATENTSCHRIFT**

(45) Veröffentlichungstag und Bekanntmachung des Hinweises auf die Patenterteilung:
**11.02.1998 Patentblatt 1998/07**

(51) Int Cl.[6]: **G01N 33/543**, G01N 33/68

(21) Anmeldenummer: **93104573.6**

(22) Anmeldetag: **19.03.1993**

(54) **Verfahren zur immunologischen Bestimmung eines oder mehrerer Analyten**

Method for the immunological determination of one or more analytes

Procédé pour la détermination immunologique d'un ou plusieurs analytes

(84) Benannte Vertragsstaaten:
AT BE CH DE DK ES FR GB IT LI LU NL PT SE

(30) Priorität: **16.04.1992 DE 4212706**

(43) Veröffentlichungstag der Anmeldung:
**20.10.1993 Patentblatt 1993/42**

(73) Patentinhaber: **Behring Diagnostics GmbH**
**35001 Marburg (DE)**

(72) Erfinder:
• **Auerbach, Bernhard**
**W-3550 Marburg (DE)**
• **Peters, Helmut**
**W-3550 Marburg (DE)**

(56) Entgegenhaltungen:
**EP-A- 0 443 599**

• **JOURNAL OF IMMUNOLOGY Bd. 138, Nr. 12, 15. Juni 1987, BALTIMORE US Seiten 4402 - 4407 FINNE ET AL. 'An IgG monoclonal antibody to group B meningococci cross- reacts with ...'**
• **PROCEEDINGS OF THE NATIONAL ACADEMY OF SCIENCES OF USA Bd. 82, Nr. 4, Februar 1985, WASHINGTON US Seiten 1194 - 1198 FROSCH ET AL. 'NZB mouse system for production of monoclonal antibodies to weak bacterial antigens: ....'**
• **JOURNAL OF NEUROCHEMISTRY Bd. 41, 1983, NEW YORK, NY Seiten 363 - 366 IBSEN ET AL. 'Quantification of the D2-glycoprotein in amniotic fluid and serum .....'**

**Beschreibung**

Die Erfindung betrifft ein verbessertes diagnostisches Verfahren zur immunologischen Bestimmung eines oder mehrerer Analyten mittels spezifischer Bindungspartner, wobei der eine spezifische Bindungspartner an einem Träger immobilisiert ist und das Ausmaß der Bindung des Analyten an den ersten spezifischen Bindungspartner mittels eines zweiten für den Analyten spezifischen Bindungspartners, der entweder direkt oder über weitere Bindungspartner markiert ist, bestimmt wird.

Das "Neural Cell Adhesion Molecule" (NCAM) ist ein interzelluläres Adhäsionsprotein, das in verschiedenen, vor allem neuralen Geweben des Menschen aber auch von Tieren, wie z.B. Mäusen, Hühnern etc., exprimiert wird. Es wurden drei Isoformen mit unterschiedlichem Molekulargewicht beschrieben, die auch noch zusätzlich, z.B. durch unterschiedliche Glycosylierungsmuster weiter modifiziert sein können (Nybroe et al., Neurochem. Int., 12: 215-262, 1988). Aus immunhistochemischen Studien ist NCAM außerdem als ein tumorassoziiertes Antigen des humanen, kleinzelligen Lungenkarzinoms (SCLC, small cell lung cancer) (Kibbelaar et al., J. Pathol. 159: 23-28, 1989) bekannt. Mit einem Immunoassay, der aufgrund der Verwendung des für $\alpha$-2,8-verknüpfte N-Acetyl-Neuraminsäureketten (2,8-NAcN) spezifischen MAK 735 (Frosch et al., Proc Natl. Acad. Sci. 82: 1194-1198, 1985; Finne et al., J. Immunol. 138: 4402-4407, 1987) als Festphasenantikörper in Kombination mit dem MAK BW SCLC-1 (ECACC Deposit Nr. 90 022 110) bzw. BW SCLC-2 (ECACC Deposit Nr. 90 022 109) die sogenannte "embryonale" NCAM-Isoform erkennt, konnte gezeigt werden, daß überraschenderweise diese Isoform bzw. ein durch die oben beschriebenen Antikörper definiertes Antigen (T-NCAM) im Serum einer großen Anzahl von SCLC-Patienten in erhöhter Konzentration vorkommt (EP-A-0 443 599 A2).

Zur Schwangerschaftsüberwachung werden verschiedene diagnostische Verfahren eingesetzt um frühzeitig fetale Mißbildungen entdecken zu können. Bewährt hat sich dabei u.a. die immunchemische Bestimmung von Alpha-Fetoprotein (AFP) im Fruchtwasser bzw. mütterlichen Serum. Erhöhte AFP-Konzentrationen weisen dabei auf einen Neuralrohrdefekt beim Embryo hin.

Aufgrund falsch positiver wie falsch negativer Befunde wird aber empfohlen, für diese Indikation zusätzliche Untersuchungen durchzuführen. Durch die Arbeit von Ibsen et al. (J. Neurochem. 41: 363-366, 1983) ist weiterhin bekannt, daß eine erhöhte Fruchtwasserkonzentration des Neural cell adhesion molecules (= D2) ein Indiz für fetale Mißbildungen ist.

Der Arbeit von Ibsen et al. ist allerdings auch zu entnehmen, daß die diagnostische Trennschärfe ihres D2-Testes, d.h. die Möglichkeit zwischen gesunden und pathologischen Proben unterscheiden zu können, mit mütterlichem Serum als Probenmaterial, das für eine erste Testung im Rahmen einer Schwangerschaftsüberwachung leichter zugänglich ist, nur unzureichend ist.

Zusätzlich zeigte sich, daß bei der Umstellung des T-NCAM-Testes von einer Mikrotitrationsplattenversion auf eine Röhrchenversion die T-NCAM-Konzentration in Seren gesunder Blutspender höher gefunden wurde, während die T-NCAM-Konzentration in Seren von Tumorpatienten in beiden Testen gleich hoch ermittelt wurde. Die Ursache hierfür kann eine in den Proben enthaltene NCAM-Isoform mit anderen Polysialinsäureketten sein, die mit dem Festphasenantikörper sowie den Tracer/Konjugatantikörpern kreuzreagiert und die mit der immunchemisch etwas reaktiveren Röhrchen-Festphase besser zu binden vermag.

Aufgabe der vorliegenden Erfindung war es daher, einen verbesserten T-NCAM-Test, bevorzugterweise für die Tumordiagnostik und Schwangerschaftsüberwachung, zu entwickeln, bei dem die diagnostische Trennschärfe, d.h. die Differenzierung in pathologische und normale Proben, verbessert ist.

Überraschenderweise zeigte sich, daß durch die Zugabe geeigneter Mengen von Substanzen, die (wie z.B. das Kapselpolysaccarid von E. coli K1) $\alpha$-2,8-verknüpfte N-Acetyl-Neuraminsäure-Ketten enthalten und spezifisch vom "Festphasenantikörper" - z. B. den im T-NCAM-Test verwendeten MAK 735 - nicht aber von den markierten "Tracer/Konjugatantikörpern" - z. B. im T-NCAM-Test verwendeten MAK BW SCLC-1 bzw. -2 - gebunden werden, zum Probeninkubationspuffer diese Aufgabe gelöst werden konnte.

Gegenstand der Erfindung ist somit ein Verfahren zur immunologischen Bestimmung eines oder mehrerer Analyten mittels spezifischer Bindungspartner, wobei der eine spezifische Bindungspartner an einem Träger immobilisiert ist und das Ausmaß der Bindung des Analyten an den ersten spezifischen Bindungspartner mittels eines zweiten für den Analyten spezifischen Bindungspartners, der entweder direkt oder über weitere Bindungspartner markiert ist, bestimmt wird, wobei die Reaktion mindestens mit einem der spezifischen Bindungspartner in Gegenwart einer Suppressorsubstanz erfolgt, die eine hohe Affinität zu diesem spezifischen Bindungspartner, nicht aber für den anderen oder einen der weiteren spezifischen Bindungspartner aufweist.

Bevorzugt ist ein solches Verfahren, in dem der erste spezifische Bindungspartner spezifisch $\alpha$-2,8-verknüpfte N-Acetyl-Neuraminsäureketten zu binden vermag und der zweite spezifische Bindungspartner für dasselbe Epitop spezifisch ist, wie die monoklonalen Antikörper BW SCLC-1 und BW SCLC-2.

Gegenstand der Erfindung ist ferner ein solches Verfahren, wobei der Bindungspartner, zu dem die Suppressorsubstanz eine Affinität hat, an die Festphase gebunden ist.

Die dabei verwendete Suppressorsubstanz enthält bevorzugterweise $\alpha$-2,8-verknüpfte N-Acetyl-Neuraminsäure-Ketten.

Besonders bevorzugterweise besteht die Suppressorsubstanz aus Komponenten des Kapselpolysaccharids von E. coli K 1 bzw. Meningokokken Typ B oder aus Colominic Acid, ganz bevorzugterweise aus Colominic Acid.

Die Suppressorsubstanz wird in einer solchen Konzentration eingesetzt, daß das Meßsignal des Teststandards um nicht mehr als 50 % inhibiert wird, bevorzugterweise um 20 bis 40 %, ganz bevorzugterweise um etwa 30 %.

In einem solchen Verfahren wird Colominic Acid in einer Konzentration von 0,01 bis 10 µg/ml eingesetzt, bevorzugterweise von 0,1 bis 1 µg/ml.

Eine mögliche Wirkungsweise dieser Substanzen ist die folgende: die 2,8-NAcN, die nicht von den Tracer/Konjugatantikörpern erkannt werden, kompetieren vermutlich mit den T-NCAM-Molekülen und der "kreuzreagierenden Substanz" um die freien Bindungsstellen des MAK 735. Aufgrund der unterschiedlichen Bindung von T-NCAM und 2,8-NAcN, z. B. an den MAK 735, werden weniger Moleküle der "kreuzreagierenden Substanz", die eine geringere Affinität zum Festphasenantikörper haben als T-NCAM oder 2,8-NAcN, gebunden und damit wird letztendlich die diagnostische Trennschärfe des T-NCAM-Testes erheblich verbessert. Entscheidend für die Auswahl von 2,8-NAcN ist deren Bindefähigkeit mit dem Festphasenantikörper. Andere Glycoproteine, die nicht spezifisch vom MAK 735 gebunden werden, aber einen erhöhten Sialinsäuregehalt aufweisen, wie z.B. Fetuin (Sigma), Mucine (Type I-S, Sigma), al-saures Glycoprotein (Behringwerke), oder Sialinsäuren (z.B. Type VI oder Type VIII, Sigma) blieben auch bei einer Konzentration bis 0.1 mg/ml unwirksam.

Das für den T-NCAM-Test gefundene Verfahren läßt sich in analoger Weise auch für andere Teste einsetzen, z.B für Antikörper-Antigen-Antikörper-"Sandwich"-Immunoassays bzw. für Antigen-Antikörper-Antigen-"Sandwich"-Immunoassays.

Hierbei wird die Bindung einer in den Proben enthaltenen kreuzreaktiven Substanz, die von beiden im Test eingesetzten spezifischen Rezeptoren R1 und R2 erkannt wird, an einen der spezifischen Rezeptoren R1, vorzugsweise dem Festphasen-gebundenen, dadurch verhindert, daß dem Probeninkubationsmedium eine Suppressorsubstanz in geeigneter Konzentration zugesetzt wird, die dadurch gekennzeichnet ist, daß sie kompetitiv die Bindung der kreuzreaktiven Substanz an R1 inhibiert und keine spezifische Bindungsstelle für den Rezeptor R2 aufweist.

Eine ähnliche Verbesserung wie mit den sehr geringen Mengen einer spezifischen Suppressorsubstanz konnte erst mit sehr viel höheren Konzentrationen von Salzen wie NaCl oder $CaCl_2$ erzielt werden. So konnte bei Verwendung eines Probeninkubationspuffers mit einer NaCl-Konzentration von mehr als 150 bis 2000 mmol/l, besonders im Bereich von 250 bis 1000 mmol/l gute Ergebnisse erzielt werden. Als Puffer haben sich als besonders geeignet erwiesen Carbonatpuffer mit einem pH > 8.0 (besonders gut pH 8 - 10) und TRIS- oder Phosphatpuffer im Bereich von pH 6 - 8.

Durch die oben beschriebenen Maßnahmen konnte die diagnostische Sensitivität des T-NCAM-Testes deutlich gesteigert werden.

Die folgenden Beispiele dienen der Erläuterung der Erfindung und schränken sie in keiner Weise ein.

**Beispiel 1:**

**Enzymimmunoassay zur T-NCAM-Bestimmung in humanen Körperflüssigkeiten**

Zur Bestimmung der Konzentration von T-NCAM wurden je 10 µl Probenmaterial sowie 100 µl Probenpuffer (OSND, Behringwerke) in die mit dem MAK 735 nach einem dem Fachmann bekannten Verfahren beschichteten Vertiefungen von Mikrotitrationsplatten (Nunc) pipettiert und eine Stunde bei 37°C inkubiert.

Nach dreimaligem Waschen mit dem verdünnten Enzygnost-Waschpuffer (OSEW, Behringwerke) wurden in die einzelnen Vertiefungen je 100 µl des MAK BW SCLC-1-POD bzw. BW SCLC-2-POD-Konjugates eingefüllt (POD = Meerrettichperoxidase). Der folgende einstündige Inkubationsschritt bei + 37° C wurde mit einem dreimaligen Waschzyklus abgeschlossen.

Für den 3. Inkubationsschritt bei Raumtemperatur wurden anschließend je 100 µl einer Puffer/Substrat-Chromogen-Lösung ($H_2O_2$/TMB; OUVG/OUVF, BW) in die Vertiefungen pipettiert und die Enzymreaktion nach 30 Minuten mit Enzygnost-Stopplösung (OSFA, BW) beendet. Die Extinktionsmessung der Proben wurde bei 450 nm durchgeführt. Anhand einer mitgeführten Standardreihe konnten die T-NCAM-Konzentrationen der Proben quantitativ ermittelt werden (Maßeinheit: U/ml).

**Beispiel 2:**

**Diagnostisches Verfahren zur Schwangerschaftsüberwachung**

Die Konzentration von T-NCAM in Fruchtwasserproben wurde analog dem im Beispiel 1 beschriebenen immunchemischen Verfahren bestimmt. Die AFP-Konzentration wurde mit einem kommerziell erhältlichen Test bestimmt.

Ergebnis: In Fruchtwasserproben wurde beim Vorhandensein embryonaler Mißbildungen (z.B. Spina bifida oder Anencephalie) erhöhte T-NCAM-Konzentrationen gemessen (Tab.1: Proben 52, 56 u. 159). Bei den Proben 53 und 139 mit pathologisch erhöhter AFP-Konzentration liegt die NCAM-Konzentration entsprechend dem klinischen Befund eines gesunden Embryos richtig im Normalbereich. Hieraus muß gefolgert werden, daß sich ein diagnostisches Verfahren zur Bestimmung von T-NCAM bzw. NCAM für die Schwangerschaftsüberwachung (Früherkennung embryonaler Mißbildungen) eignet.

Tab.1:

| T-NCAM-Konzentration in Fruchtwasserproben | | | | |
|---|---|---|---|---|
| Proben Nr. | Schwangerschaftswoche | AFP (µg/ml) | T-NCAM (U/ml) | Diagnose |
| 52 | 16 | 188 | 52.7 | Anencephalus |
| 56 | 16 | 556 | 57.2 | Anencephalus |
| 159 | 20-21 | 76 | 8.9 | Spina bifida |
| 53 | 16 | 92 | 1.6 | Gesundes Kind |
| 139 | 19 | 200 | 5.1 | " |
| 12 | 16 | 27 | 1.5 | " |
| 13 | 16 | 30 | 1.5 | " |
| 14 | 16 | 24 | 3.7 | " |
| 15 | 16 | 30 | 1.8 | " |
| 16 | 16 | 23 | 3.5 | " |

**Beispiel 3:**

**Chemolumineszenzimmunoassay zur T-NCAM-Bestimmung in humanen Körperflüssigkeiten**

Zur Bestimmung der Konzentration von T-NCAM wurden je 20 µl Probenmaterial sowie 200 µl Probenpuffer (z.B. OSND, Behringwerke) in die mit dem MAK 735 nach einem dem Fachmann bekannten Verfahren beschichteten Polystyrolröhrchen (Greiner) pipettiert und eine Stunde bei Raumtemperatur und unter Schütteln inkubiert.

Nach zweimaligem Waschen mit je 2 ml BeriLux-Waschpuffer (Behringwerke) wurden in die einzelnen Vertiefungen je 200 µl einer Lösung des mit Acridinium-N-acylsulfonamidgelabelten MAK BW SCLC-1 bzw. BW SCLC-2 eingefüllt.

Der folgende einstündige Inkubationsschritt bei Raumtemperatur und unter Schütteln wurde mit einem dreimaligen Waschzyklus abgeschlossen.

Anschließend wurden in einem Luminometer automatisch je 300 µl BeriLux Analyzer-Reagenz R1 und R2 (Behringwerke) in die Röhrchen eingefüllt und das luminogene Meßsignal über einen Zeitraum von 3 Sekunden gemessen. Anhand einer mitgeführten Standardreihe konnten die T-NCAM-Konzentrationen der Proben quantitativ ermittelt werden (Maßeinheit: U/ml).

**Beispiel 4:**

**Zusatz von 2,8-NAcN zum Probeninkubationspuffer zur Verbesserung der Normalseren/Tumorseren-Diskriminierung**

Die T-NCAM-Konzentrationen von Seren gesunder Blutspender oder von T-NCAM-haltigen Proben, z.B. von Patienten mit kleinzelligen Lungenkarzinom, wurden mit dem immunchemischen Testverfahren gemäß Beispiel 3 ermittelt. Hierbei wurde das Probeninkubationsmedium (OSND, Behringwerke) mit verschiedenen Substanzen versetzt, unter anderem mit Verbindungen wie z.B. mit einer Präparation des Kapselpolysaccharids von E. coli K 1 (s.a. Rohr & Troy, J. Biol Chem. 255: 2332-2342, 1980; Weisgerber & Troy, J. Biol Chem. 265: 1578-1587, 1990) oder Colominic Acid (Sigma)), die wie z.B. auch das Kapselpolysaccarid der Meningokokken Typ B α-2,8-verknüpfte N-Acetyl-Neuraminsäure-Ketten tragen und spezifisch vom MAK 735 nicht aber von den MAK BW-SCLC-1 oder -2 gebunden werden. Die zugegebene Menge an 2,8-NAcN wurde so ausgewählt, daß das Meßsignal der Teststandards zwischen 1 und 50% inhibiert wurde. Andere Glycoproteine mit einem erhöhten Sialinsäuregehalt, wie z.B. Fetuin (Sigma), Mucine (Type I-S, Sigma), α1-saures Glycoprotein (Behringwerke), oder Sialinsäuren (z.B. Type VI oder Type VIII, Sigma) wurden bis zu einer Konzentration von 0.1 mg/ml eingesetzt.

Ergebnis: Nur durch den Zusatz von 2,8-NAcN konnte eine verbesserte Tumorseren/Normalseren-Diskriminierung erzielt werden (s.a. Beispiel in Tab.2 u. Figur).

Tab.2:

| T-NCAM-Bestimmung in Normalseren und Tumorseren unter Verwendung eines 2,8-NAcN-haltigen Probenpuffers (Zusatz von E.coli K 1-Kapselpolysaccharid) | | |
|---|---|---|
| Probenpuffer | | |
| ohne 2,8-NAcN | | plus 2,8-NAcN |
| Tumorseren | T-NCAM (U/ml) | T-NCAM (U/ml) |
| TS 53 | 76.4 | 80.3 |
| TS 608 | 70.9 | 58.4 |
| TS 1058 | 102.0 | 104.7 |
| TS 1350 | 36.7 | 46.5 |
| TS 1462 | 91.9 | 88.9 |
| Normalseren | | |
| NS 1 | 16.6 | 7.9 |
| NS 2 | 21.7 | 11.2 |
| NS 3 | 10.7 | 5.4 |
| NS 4 | 17.6 | 9.6 |
| NS 5 | 14.3 | 6.7 |
| NS 6 | 11.7 | 7.2 |
| NS 7 | 13.5 | 8.2 |
| NS 8 | 9.4 | 5.0 |
| NS 9 | 10.2 | 4.7 |
| NS 10 | 10.4 | 7.6 |
| MW: | 13.6 | 7.3 |
| SD: | 3.8 | 2.0 |

**Beispiel 5:**

**Zusatz von Salzen/Ionen zum Probeninkubationspuffer zur Verbesserung der Normalseren/Tumorseren-Diskriminierung**

Die T-NCAM-Konzentrationen von Seren gesunder Blutspender oder von T-NCAM-haltigen Proben, z.B. von Patienten mit kleinzelligem Lungenkarzinom, wurden mit dem immunchemischen Testverfahren gemäß Beispiel 3 ermittelt. Hierbei wurde ein 50 mM Tris/HCl-Puffer, pH 7.0, der desweiteren 0.1 % Humanserumalbumin und 0.5 % Tween 20 enthielt, mit verschiedenen Salzen versetzt und als Probeninkubationspuffer eingesetzt (Ergebnisse siehe Tab. 3).

Tab.3:

| T-NCAM-Bestimmung in Normalseren und T-NCAM-haltigen Proben | | | |
|---|---|---|---|
| Probenpuffer mit | 0 mM NaCl | 250 mM NaCl | 500 mM NaCl |
| (T-NCAM in U/ml) | | | |
| | T-NCAM-Proben: | | |
| A | 62.7 | 78.6 | 73.4 |
| B | 71.6 | 81.6 | 75.9 |
| C | 66.2 | 73.0 | 66.5 |
| D | 63.9 | 77.9 | 78.9 |

EP 0 565 903 B1

Tab.3: (fortgesetzt)

| T-NCAM-Bestimmung in Normalseren und T-NCAM-haltigen Proben | | |
|---|---|---|
| Probenpuffer mit 0 mM NaCl | 250 mM NaCl | 500 mM NaCl |
| (T-NCAM in U/ml) | | |
| T-NCAM-Proben: | | |
| E  77.6 | 80.8 | 72.5 |
| | | |
| Normalseren: | | |
| | | |
| NS 1  18.8 | 12.9 | 9.2 |
| NS 2  23.1 | 17.3 | 10.9 |
| NS 3  10.9 | 7.0 | 5.6 |
| NS 4  17.7 | 13.4 | 10.0 |
| NS 5  18.3 | 9.7 | 7.1 |
| NS 6  11.1 | 8.4 | 6.8 |
| NS 7  14.9 | 11.1 | 7.9 |
| NS 8  9.9 | 6.8 | 5.3 |
| NS 9  13.7 | 7.8 | 5.1 |
| NS 10  15.7 | 10.9 | 6.4 |
| | | |
| MW:  15.4 | 10.5 | 7.4 |
| SD:  4.0 | 3.2 | 1.9 |

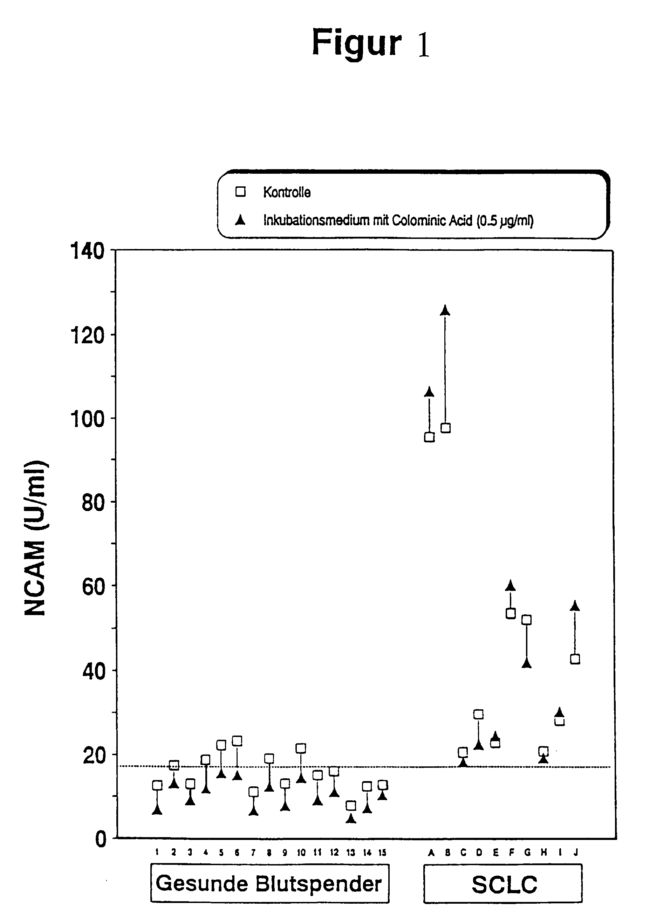

**Beschreibung der Figur**

**Figur:** T-NCAM-Serumkonzentration von gesunden Blutspendern und Patienten mit kleinzelligem Lungenkarzinom (SCLC) bestimmt mit einem T-NCAM-Test unter Verwendung eines Probeninkubationsmediums (OSND, Behringwerke) mit und ohne (Kontrolle) Zusatz von 0,5 µg/ml Colominic Acid (Sigma).

**Patentansprüche**

1. Verfahren zur immunologischen Bestimmung eines oder mehrerer Analyten mittels spezifischer Bindungspartner, wobei der eine spezifische Bindungspartner an einem Träger immobilisiert ist und das Ausmaß der Bindung des Analyten an den ersten spezifischen Bindungspartner mittels eines zweiten für den Analyten spezifischen Bindungspartners, der entweder direkt oder über weitere Bindungspartner markiert ist, bestimmt wird, dadurch gekennzeichnet, daß die Reaktion mit mindestens einem der spezifischen Bindungspartner in Gegenwart einer Suppressorsubstanz erfolgt, die eine hohe Affinität zu diesem spezifischen Bindungspartner, nicht aber für den anderen oder einen der weiteren spezifischen Bindungspartner aufweist.

2. Verfahren nach Anspruch 1 dadurch gekennzeichnet, daß der erste spezifische Bindungspartner spezifisch $\alpha$-2,8-verknüpfte N-Acetyl-Neuraminsäureketten zu binden vermag und der zweite spezifische Bindungspartner für dasselbe Epitop spezifisch ist, wie die monoklonalen Antikörper BW SCLC-1 ECACC Hinterlegungs-Nr. 90 022 110 und BW SCLC-2 ECACC Hinterlegungs-Nr. 90 022 109.

3. Verfahren nach Anspruch 1 oder 2 dadurch gekennzeichnet, daß der Bindungspartner, zu dem die Suppressorsubstanz eine Affinität hat, an die Festphase gebunden ist.

4. Verfahren nach Anspruch 1, 2 oder 3 dadurch gekennzeichnet, daß die Suppressorsubstanz $\alpha$-2,8-verknüpfte N-Acetyl-Neuraminsäure-Ketten enthält.

5. Verfahren nach Anspruch 1, 2 oder 3 dadurch gekennzeichnet, daß die Suppressorsubstanz aus Komponenten des Kapselpolysaccharids von E. coli K 1 bzw. Meningokokken Typ B oder aus Colominic Acid, bevorzugterweise aus Colominic Acid besteht.

6.  Verfahren nach Anspruch 1, 2 oder 3 dadurch gekennzeichnet, daß die Suppressorsubstanz in einer solchen Konzentration eingesetzt wird, daß das Meßsignal des Teststandards um nicht mehr als 50 % inhibiert wird.

7.  Verfahren nach Anspruch 1, 2 oder 3 dadurch gekennzeichnet, daß Colominic Acid in einer Konzentration von 0,01 bis 10 µg/ml eingesetzt wird.

## Claims

1.  A method for the immunological determination of one or more analytes by means of specific binding partners, the one specific binding partner being immobilized on a carrier and the extent of the binding of the analyte to the first specific binding partner being determined by means of a second binding partner which is specific for the analyte and which is labeled either directly or via further binding partners, wherein the reaction with at least one of the specific binding partners takes place in the presence of a suppressor substance which has a high affinity for this specific binding partner but not for the other or one of the further specific binding partners.

2.  The method as claimed in claim 1, wherein the first specific binding partner is able specifically to bind $\alpha$-2,8-linked N-acetylneuraminic acid chains, and the second specific binding partner is specific for the same epitope as the monoclonal antibodies BW SCLC-1 ECACC deposition No. 90 022 110 and BW SCLC-2 ECACC deposition No. 90 022 109.

3.  The method as claimed in claim 1 or 2, wherein the binding partner for which the suppressor substance has an affinity is bound to the solid phase.

4.  The method as claimed in claim 1, 2 or 3, wherein the suppressor substance contains $\alpha$-2,8-linked N-acetylneuraminic acid chains.

5.  The method as claimed in claim 1, 2 or 3, wherein the suppressor substance is composed of components of the capsule polysaccharide from E. coli K 1 or type B meningococci or of colominic acid, preferably of colominic acid.

6.  The method as claimed in claim 1, 2 or 3, wherein the suppressor substance is employed at a concentration such that the measurement signal of the test standard is inhibited by not more than 50%.

7.  The method as claimed in claim 1, 2 or 3, wherein colominic acid is employed at a concentration of 0.01 to 10 µg/ml.

## Revendications

1.  Procédé pour la détermination immunologique d'un ou plusieurs analytes au moyen de partenaires de liaison spécifiques, un partenaire de liaison spécifique étant immobilisé sur un support et le degré de la liaison de l'analyte au premier partenaire de liaison spécifique étant déterminé au moyen d'un second partenaire de liaison spécifique de l'analyte, qui est marqué soit directement, soit par l'intermédiaire d'autres partenaires de liaison, caractérisé en ce que la réaction est effectuée avec au moins un partenaire de liaison spécifique en présence d'une substance suppresseur, qui présente une haute affinité pour ce partenaire de liaison spécifique, mais non pour l'autre ou l'un des autres partenaires de liaison spécifiques.

2.  Procédé selon la revendication 1, caractérisé en ce que le premier partenaire de liaison spécifique permet la liaison spécifique de chaînes d'acide N-acétylneuraminique reliées en $\alpha$-2,8 et le second partenaire de liaison spécifique est spécifique du même épitope que les anticorps monoclonaux BW SCLC-1 (n° de dépôt ECACC 90 022 110) et BW SCLC-2 (n° de dépôt ECACC 90 022 109).

3.  Procédé selon la revendication 1 ou 2, caractérisé en ce que le partenaire de liaison pour lequel la substance suppresseur a une affinité est fixé sur la phase solide.

4.  Procédé selon la revendication 1, 2 ou 3, caractérisé en ce que la substance suppresseur contient des chaînes d'acide N-acétylneuraminique reliées en $\alpha$-2,8.

5. Procédé selon la revendication 1, 2 ou 3, caractérisé en ce que la substance suppresseur consiste en composants du polysaccharide de capsule de *E. coli* K1 ou de méningocoques de type B ou en acide colominique, de préférence en acide colominique.

6. Procédé selon la revendication 1, 2 ou 3, caractérisé en ce que la substance suppresseur est utilisée à une concentration telle que le signal de mesure de la substance de référence utilisée dans l'essai n'est pas inhibée à plus de 50 %.

7. Procédé selon la revendication 1, 2 ou 3, caractérisé en ce que l'acide colominique est utilisé à une concentration de 0,01 à 10 μg/ml.

EP 0 565 903 B1

# **Figur** 1

9